(19) Europäisches Patentamt
European Patent Office
Office européen des brevets

(11) **EP 2 142 467 B2**

(12) **NEW EUROPEAN PATENT SPECIFICATION**
After opposition procedure

(45) Date of publication and mention
of the opposition decision:
**10.12.2014 Bulletin 2014/50**

(45) Mention of the grant of the patent:
**22.12.2010 Bulletin 2010/51**

(21) Application number: **08748858.1**

(22) Date of filing: **02.04.2008**

(51) Int Cl.:
**C01B 3/38** (2006.01)     **C07C 29/151** (2006.01)

(86) International application number:
**PCT/EP2008/002621**

(87) International publication number:
**WO 2008/122399 (16.10.2008 Gazette 2008/42)**

(54) **COMBINED REFORMING PROCESS FOR METHANOL PRODUCTION**

KOMBINIERTES REFORMIERUNGSVERFAHREN FÜR DIE METHANOLHERSTELLUNG

PROCÉDÉ DE REFORMAGE COMBINÉ POUR LA PRODUCTION DE MÉTHANOL

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **04.04.2007 EP 07007039**

(43) Date of publication of application:
**13.01.2010 Bulletin 2010/02**

(73) Proprietor: **Saudi Basic Industries Corporation
11422 Riyadh (SA)**

(72) Inventors:
• **AHMED, Ijaz
11551 Riyadh (SA)**
• **BASHIR, Mubarak
11551 Riyadh (SA)**

• **AL-OTAIBI, Metab
11551 Riyadh (SA)**

(74) Representative: **Modiano, Micaela Nadia et al
Modiano & Partners
Thierschstrasse 11
80538 München (DE)**

(56) References cited:
**EP-A1- 0 959 120     EP-A2- 0 440 258
EP-A2- 0 522 744     EP-A2- 0 989 094
CN-A- 1 702 038     GB-A- 2 407 819
US-A- 4 888 130     US-A- 6 100 303**

• **T.S. CHRISTENSEN: 'Adiabatic prereforming of
hydrocarbons- an important step in syngas
production' APPLIED CATALYSIS ELSEVIER vol.
138, 01 January 1996 - 31 December 1996, pages
285 - 309**

## Description

The invention relates to a process for making methanol from a methane-rich gaseous feedstock comprising a combined reforming process for making a synthesis gas mixture from a desulphurised methane-rich gaseous feedstock, wherein a combination of different reforming reactors is used.

Such a combined reforming process is known from patent publication US 6100303. In this document a process for making synthesis gas for subsequent use in methanol production is described, wherein a desulphurised feedstock gas consisting of a hydrocarbon gas having an atomic ratio of 3-4, for example natural gas composed mainly of methane, is reformed using a combination of 3 different reforming reactors. The feedstock is first mixed with steam and then fed to a combustion-type (also called fired) steam methane reforming reactor (steam methane reformer, hereinafter abbreviated as SMR) and a heat-exchange type steam reforming reactor that is heated with hot gasses produced elsewhere in the process (also called gas heated reformer, hereinafter abbreviated as GHR), which two reactors are operated in parallel arrangement. The effluent gasses from SMR and GHR are mixed and fed to a secondary reforming unit together with oxygen, wherein the gasses undergo a catalytic partial oxidation reaction under essentially adiabatic conditions in addition to further reaction with steam. This reforming reactor is also referred to as auto-thermal reformer (abbreviated as ATR), as the excess heat generated by exothermic reaction is used to supply heat for the endothermic steam reforming reaction. The SMR unit is heated by burning part of the hydrocarbon feedstock gas and a purge of synthesis gas. The feed ratio of feedstock gas to the SMR and GHR units can vary from 1-3 to 3-1.

In the past decades, numerous processes have been developed to produce synthesis gas (also referred to in short as syngas) as one of the most important feedstocks in chemical industry. Syngas is a gaseous mixture containing hydrogen ($H_2$) and carbon monoxide (CO), which may further contain other gas components like carbon dioxide ($CO_2$), water ($H_2O$), methane ($CH_4$), and nitrogen ($N_2$). Natural gas and (light) hydrocarbons are the predominant starting material for making synthesis gas. Syngas is successfully used as synthetic fuel and also in a number of chemical processes, such as synthesis of methanol or ammonia, Fischer-Tropsch type and other olefin syntheses, hydroformulation or carbonylation reactions (oxo processes), reduction of iron oxides in steel production, etc. The composition of synthesis gas, and thus its suitability for subsequent use for e.g. methanol production, is characterized mainly by its hydrogen and carbon monoxide content; generally presented by the so-called stoichiometric number (SN), which is defined as

$$SN = ([H_2]-[CO_2]) / ([CO]+[CO_2])$$

wherein the concentrations of components are expressed in vol% or mol%.

The value of SN is highly dependent on the reforming process technology used to make syngas. An overview of different technologies and their advantages and limitations is for example given by P. F. van den Oosterkamp in chapter "Synthesis Gas Generation: Industrial" of the "Encyclopedia of Catatysis" (John Wiley & Sons; posted on-line 2002/12/13, available via DOI: 10.1002/0471227617.eoc196).

The conventional technology for producing syngas from a methane feedstock is the reaction with water (steam) at high temperatures, generally called hydrocarbon steam reforming.

If a feedstock is used in a reforming process that is rich in higher hydrocarbons, like naphtha, the feedstock first needs to be treated in a so-called pre-reforming step, in order to convert the heavy hydrocarbons in the feed into methane, hydrogen and carbon oxides. Such higher hydrocarbons are more reactive than methane in steam reforming, and would -if present in the feed- lead to carbon formation and thus to deactivation of the catalyst employed in steam reforming. In such a pre-reformer several reactions take place simultaneously; the most important being hydrocarbon steam reforming (1), water gas shift (2), and methanation (3) reactions, which can be represented, respectively, as:

$$(1) \qquad C_nH_m + n\,H_2O \; \leftrightarrows \; n\,CO \; + \; (m/2 + n)\,H_2$$

$$(2) \qquad CO + H_2O \leftrightarrows CO_2 + H_2$$

$$(3) \qquad CO + 3\,H_2 \leftrightarrows CH_4 + H_2O$$
$$CO_2 + 4\,H_2 \leftrightarrows CH_4 + 2\,H_2O$$

Such a pre-reformer is typically operated adiabatically at temperatures between 320 and 550 °C, and is generally referred to as an adiabatic pre-reformer (hereinafter abbreviated as APR).

In a steam methane reformer (SMR) methane-rich gas is converted into a mixture containing carbon monoxide, carbon dioxide, hydrogen and unreacted methane and water in the so-called steam reforming (4) and carbon dioxide reforming (5) reactions, represented as:

$$(4) \qquad CH_4 + H_2O \ \rightleftharpoons \ CO \ + \ 3\,H_2$$

$$(5) \qquad CH_4 + CO_2 \ \rightleftharpoons \ CO \ + \ 2\,H_2$$

These reforming reactions are strongly endothermic, while the accompanying water shift reaction is moderately exothermic. Such process thus calls for a reactor wherein heat management is extremely important. For the steam reforming process, several types of reactors are possible, such as the conventional widely used top-fired or side-fired reformers. In practice, a SMR unit may contain from 40 up to 1000 tubes, each typically 6-12 m long, 70-160 mm in diameter, and 10-20 mm in wall thickness. These tubes are vertically placed in a rectangular furnace or firebox, the socalled radiant section. The reactor tubes contain nickel-based catalyst, usually in the form of small cylinders or rings. The reactor tubes are fired by burners, which may be located at the bottom, at the side, or at the top of the furnace. Combustion of the fuel takes place in the radiant section of the furnace. After the flue gas has supplied its heat to all the reactor tubes, it passes into the convection section where it is further cooled by heating other streams such as process feed, combustion air, and boiler feed water as well as producing steam. The product gas, typically leaving the reformer at a temperature of 850-950°C, is cooled in a process gas waste heat boiler to produce process steam for the reformer. The syngas made with conventional steam reforming typically has a SN of between 2.6 and 2.9. For methanol production a composition having SN coming close to the theoretical value of 2 is preferred. The SN value of the syngas composition can be lowered by for example adding carbon dioxide; or by combined reforming (see below).

Steam reforming can also be performed in reactors wherein the necessary heat is supplied by heat exchange rather than by direct firing, for example by convective heat transfer from hot flue gasses and/or from hot syngas produced at another stage of a process. Several reactor concepts have been developed for this purpose, the name gas heated reformer (GHR) generally being used for a reactor that makes use of the heat present in syngas that is being produced in an auto-thermal reforming unit (ATR; see below).

In an ATR conversion of a methane feedstock with oxygen (as pure oxygen, air, or enriched air) takes place in combination with the conversion with steam; an ATR is basically a combination of SMR and partial oxidation (POx) technology. In addition to the reactions mentioned above, also following strongly exothermic partial oxidation reactions (6) take place:

$$(6) \qquad \begin{aligned} CH_4 + \tfrac{1}{2}\,O_2 \ &\rightleftharpoons \ CO \ + \ 2\,H_2 \\ CH_4 + \tfrac{3}{2}\,O_2 \ &\rightleftharpoons \ CO \ + \ 2\,H_2O \end{aligned}$$

Desulphurised feedstock mixed with steam is introduced into the ATR reactor, as is oxygen in an appropriate amount. The upper part of the reactor basically consists of a burner mounted in the reactor shell. The exothermic reaction with oxygen delivers the endothermic heat of reaction of the steam reforming reaction, such that the overall reaction is auto-thermal, and takes place in the upper part; whereas the catalyzed reforming reaction takes place in a fixed bed in the lower part. Operating temperatures are relatively high, on the order of 1000°C, enabling very low amounts of unconverted methane in the product gas. The syngas produced in an ATR has a relatively low concentration of hydrogen; for subsequent methanol production mixing with hydrogen from another source will be needed.

EP 0989094 A2 indicates that also for reforming a feedstock comprising higher hydrocarbons via an ATR unit it is advantageous to first pre-reform the feedstock in an APR.

Several process schemes that combine a steam reformer and an ATR unit (or a POx reactor wherein only partial oxidation takes place), in different lay-outs have been proposed. Advantages of such combined reforming processes include controlling the SN of the syngas made at a targeted value.

As already indicated above, a combination of ATR and GHR technologies makes more efficiently use of energy; a further advantage being that adjustment of the SN of the syngas is possible; e.g. closer to the value of 2 as desired for methanol production.

WO 93/15999 describes a process of making syngas by dividing a feed gas stream over a steam reformer and a partial oxidation reactor, and feeding the combined effluent streams to a second steam reformer.

In US 4999133 a process for making syngas suitable for methanol production is disclosed, wherein a part of the feed is passed over a steam reformer, and resulting effluent and the other part of feed are fed to an ATR unit.

US 5512599 relates to a process for making methanol from syngas on a large scale and high energy efficiency, comprising a first step of steam reforming a hydrocarbon feed in a GHR reactor, followed by a partial oxidation and a second steam reforming step in an ATR unit, wherein effluent gas from ATR is used as a heat source for the GHR.

US 5496859 discloses a process for making methanol from syngas, wherein a desulphurised methane-rich feed is supplied to an ATR and a steam reformer operated in parallel, and effluent streams are combined and fed to a second ATR to result in a syngas of proper composition and pressure for subsequent methanol synthesis.

EP 0522744 A2 describes a process for making a.o. methanol from syngas, wherein a desulphurised hydrocarbon feedstock is split-up into 2 streams, of which a first stream is fed to a SMR unit, and a second stream is fed to an APR and partial oxidation reactor operated in series, followed by cooling and mixing both reformed streams.

US 2004/0063797 A1 describes a process for making syngas especially suited for subsequent use in Fischer-Tropsch synthesis, wherein a desulphurised hydrocarbon feedstock is reformed in an APR, one or more steam reformers and an ATR, which are all operated in series.

EP 1403216 A1 describes a process for making syngas especially suited for subsequent use in Fischer-Tropsch synthesis, wherein a desulphurised hydrocarbon feedstock is reformed in one or more steam reformers and an ATR, which is operated in parallel with the other reformers.

GB 2407819 A discloses a process of making syngas from a hydrocarbon, e.g. natural gas, which process employs a combination of 3 reforming units, wherein the feed first passes an APR and then is split and fed to SMR and ATR units operated in parallel; to enable high syngas production capacity.

EP 1241130 A1 discloses a process of making syngas from light natural gas, by first treating the feed in an APR unit at 500-750°C with a special catalyst, followed by conventional steam reforming; in order to reduce the heat supply required in steam reforming.

In EP 0440258 A2 a steam reforming process with improved reutilisation of heat is proposed, wherein a desulphurised hydrocarbon feed is first reacted in a first GHR, and the gas stream is then divided into 2 parallel streams, of which the first stream is fed to a SMR and the second stream to a further GHR, after which the effluent streams are combined and fed to an ATR unit.

EP 0959120 A1 discloses combined steam reforming processes aiming to optimise the energy efficiency by using heat from combustion gasses, including a scheme wherein GHR, SMR and ATR units are operated in series, and a scheme wherein the feed is fed to GHR and SMR units operated in parallel followed by reacting the combined effluent streams in an ATR.

Methanol is one the most important chemical raw materials; in 2000 about 85% of the methanol produced would be used as a starting material or solvent for synthesis, whereas its use in the fuel and energy sector has been increasing rapidly. Since the 1960's, methanol synthesis from sulphur-free syngas with Cu-based catalysts has become the major route, as it can be operated at fairly mild reaction conditions. An overview of such methanol processes can be found in for example in the chapter "Methanol" in "Ullmann's Encyclopedia of Industrial Chemistry" (Wiley-VCH Verlag; posted on-line 2000/06/15, available via DOI: 10.1002/14356007.a16_465).

Regarding the increasing demand for fuel and energy, there is a need in industry for ever larger and more efficient methanol production plants. Presently applied integrated production processes for making methanol from hydrocarbon feedstock typically have a maximum single line capacity on the order of 5000-7000 mtpd (metric ton per day). Practical limitations are encountered especially in syngas production, i.e. in the maximum size of available reforming reactors and of air separation units producing oxygen.

For example, limitations in the maximum size of a SMR unit lay in the number of tubes, even gas distribution, and in heat transfer. About 1000 tubes is considered to be the maximum for a single unit operation, otherwise it will not be possible to control uniform distribution of gasses and thus heat to all tubes. Reliability of all units is paramount, as minimizing down-time is a prerequisite for economical operation. Further capacity limitation results from a certain maximum amount of energy that can be transferred to the tubes. It is thus estimated that a technically and economically feasible SMR reactor of maximum capacity is characterized by a maximum reforming heat load of about 1150 GJ/h.

Production capacity of a GHR unit is mainly limited by a practical maximum in energy input by heat exchange with hot gasses; which is estimated to be about 420 GJ/h. Presently available ATR units do not have the above limitations of steam reformers, but the maximum production capacity in this case is in practice limited by the volume of oxygen that is available. In most cases, oxygen is to be supplied from an air separation unit (abbreviated as ASU). The maximum size of a single state-of-the-art ASU is for technical and economical reasons considered to be about 4000 mtpd (which is equivalent to about 5200 kmol/h of oxygen). The equivalent maximum methanol production capacity based on such an ATR would be about 4500-6000 mtpd.

Although integrated production processes for making methanol from hydrocarbon in practice have a maximum single line capacity of about 6000 mtpd, schemes for larger scale plants have been proposed.

In US 6100303 it is stated that based on the proposed combined reforming process scheme a methanol plant with capacity of about 10000 mtpd can be designed. A disadvantage of this process scheme, however, is that it requires very high energy input for the SMR and GHR units, exceeding the above discussed maxima, i.e. in the range 1330-2580 vs 1150 GJ/h for the SMR, and/or 1380-1410 vs 420 GJ/h for the GHR unit.

It is thus an object of the present invention to provide a combined reforming process that enables a single-line process for making methanol from a methane-rich gaseous feedstock with a capacity of at least 10000 mtpd, which reforming process uses reforming reactors and other equipment with capacities within current practical limitations.

This object is achieved according to the process as defined in claim 1. Preferred embodiments are defined in the dependent claims.

With the combined reforming process compassed by the invention it is possible to produce syngas with adjustable composition and at very high capacity in a single line. The process allows designing a methane-to-methanol production plant with a capacity of at least 10000 mtpd using technically and economically feasible reforming equipment. The process further shows high energy efficiency. A further advantage is that the process has low methane leakage, meaning that the final syngas has a low content of inerts, resulting in a high overall conversion of methane to methanol. Further, the highcapacity single-line syngas process reduces the investments required per ton of methanol production capacity.

In US 6100303 also a combined reforming process is described that applies a combination of different reactors including a pre-reforming unit, but therein it is indicated to only apply an APR unit if the feedstock is a liquid hydrocarbon having an atomic ratio H/C of 2-3, such as naphtha, and not in case of a methane-rich gaseous feedstock. In addition, this document nor other publications contain a suggestion to divide a pre-reformed gas stream to three different reforming units operated in parallel.

Within the context of the present application a methane-rich gaseous feedstock is considered to be a feedstock that contains as least 80 mol% of methane (based on total hydrocarbon content of the feedstock). A suitable example of such a feedstock is natural gas, as obtained from gas or oil fields. The primary component of natural gas is methane, which is generally present in amounts of from 80 to 97 mol%. Natural gas also contains other gaseous hydrocarbons such as ethane, typically from about 3 to 15 mol%, propane, butane and small amounts of higher hydrocarbons (generally less than 5 mol% in total), as well as sulphur-containing gases, like hydrogen sulphide, in varying amounts. Further minor (or even trace) amounts of nitrogen, helium, carbon dioxide, water, odorants, and metals like mercury can also be present. The exact composition of natural gas varies with its source.

Organosulphur compounds and hydrogen sulphide are common contaminants, which should be removed prior to use of natural gas as a feedstock in the present process, to avoid poisoning of reforming catalysts. Desulphurisation can be done with conventional techniques. In a suitable process, the natural gas is first compressed to 3-4 MPa with a centrifugal or reciprocating compressor. A hydrogen-rich stream (for example a purge stream from a methanol synthesis loop) is mixed with the natural gas, usually after compression, and hydrogen concentration in the gas is maintained at a level of 2-5 vol%. The stream is preheated to 350-380 °C and passed over an adiabatic catalytic reactor containing a hydrodesulphurisation catalyst, e.g. Co-Mo- or Ni-Mo-based. The organo-sulphur compounds in the feedstock are converted to $H_2S$, which is subsequently removed by passing over a suitable absorbent, like ZnO, in a down stream vessel. Preferably, the sulphur content of the desulphurised gaseous feed is at a level of below 1 ppm.

The desulphurised methane-rich feedstock that is used in the process according to the invention contains at least 90 mol% of methane, preferably at least 92, 94 even at least 96 mol% of methane. In the APR virtually all higher hydrocarbon present is converted into methane. In addition, also some methane steam reforming takes place, as well as water gas shift and methanation reactions (see reactions (1)-(4) above). Although an APR can be operated more efficiently the higher the content of higher hydrocarbons in the feed, it is a specific advantage of the present process to include an APR unit in the scheme, to increase overall syngas production capacity from methane. A further advantage of the APR step is, that the temperature of the pre-reformed gas stream can be heated to higher temperatures, e.g. to about 650 °C or even above, without the risk of carbon formation. A higher temperature of the gas stream entering subsequent downstream reformers further improves reaction and energy efficiency of the process.

In the process according to the invention the desulphurised methane-rich feedstock is mixed with steam, typically at a pressure of 3-4 MPa and a temperature of 350-370 °C. In this mixed feed stream a steam to carbon ratio of from 0.5 to 3.5 is maintained, and the mixed feed stream is preheated to 500-550 °C, for example with a heat exchanger. For this purpose a heat exchanger coil that is installed in the convection duct of the steam methane reformer may be advantageously used.

The preheated mixed feed stream is then passed through a conventional adiabatic pre-reformer, which typically contains a Ni-based pre-reforming catalyst. In the APR all higher hydrocarbons are converted, and also some of the methane is steam reformed to CO, $CO_2$ and $H_2$. The extent of reforming depends on various factors, such as feed preheat temperature, operating pressure, feed gas composition and steam to carbon ratio.

The design of the APR is not critical, and available designs can be applied to make a unit of suitable size for the present process.

For a methane-rich feedstock as used in the process according to the invention, the combination of above mentioned endothermic and exothermic reactions occurring in the APR may lead to a net temperature drop during the pre-reforming step, for example from an inlet temperature of about 500-550 °C to below 500 °C, depending on the content of higher hydrocarbons.

The pre-reformed gas stream coming from the APR is then heated to increase its temperature to over 550°C, preferably to a temperature of about 550-700°C, more preferably to about 600-650°C in a heat exchange coil; preferably in a coil that is placed in the convention duct of the steam methane reformer used down-stream. Heating the pre-reformed gas stream to such higher temperatures can be done in the process according to the invention with minor or even without carbon formation. A further advantage is that the higher starting temperature of inlet gas promotes conversion in subsequent reactors.

The heated pre-reformed gas stream is then divided into three streams that are fed to the reforming reactors operated in parallel; that is one stream is fed to an ATR, a second stream to a SMR, and the third stream to a GHR. The reformers may optionally also be operated partly in series. The gas distribution rates to the different reformers are depending on the actual process scheme applied.

Preferably, about 35-65 vol% of the heated pre-reformed gas stream is fed to the ATR unit in order to fully use the reactor capacity, which is limited by a maximum supply rate of oxygen of about 5200 kmol/h (based on maximum size of a single ASU). Of the remaining pre-reformed gas about 25-35 vol% is fed to the SMR, and about 5-40 vol% is fed to the GHR unit, in order to optimally utilise their capacities.

In a preferred embodiment of the process according to the invention, the heated pre-reformed gas stream is divided into three streams that are fed to ATR, SMR, and GHR reactors operated in parallel. The reformed stream leaving the ATR is first used to heat the GHR, and then mixed with the reformed streams from the SMR and GHR to form a combined syngas stream. The syngas stream is then cooled, and compressed in case of subsequent methanol synthesis steps.

Preferably, about 55-65, more preferably about 60-65 vol% of the pre-reformed gas stream is fed to the ATR, about 30-35 vol% is fed to the SMR, and about 5-15, more preferably about 5-10 vol% is fed to the GHR, to optimise process efficiency and to control composition of the syngas (with SN preferably being between 2.0 and 2.2 for subsequent use in methanol synthesis).

In another preferred embodiment of the process according to the invention, the heated pre-reformed gas stream is divided into three streams that are fed to ATR, SMR, and GHR reactors operated in parallel, with the GHR also in series with the ATR; i.e. GHR effluent is fed to the ATR. The reformed stream leaving the ATR is first used to heat the GHR, and then mixed with the reformed stream from the SMR to form a combined syngas stream. The syngas stream is then cooled, and compressed in case of subsequent methanol synthesis steps.

Preferably, about 25-30 vol% of the pre-reformed stream is fed to the SMR, about 20-40, more preferably about 25-30 vol% is fed to the GHR, and the effluent stream from the GHR and about 35-55, more preferably 45-50 vol% of the pre-reformed gas stream are mixed and fed to the ATR, to optimise process efficiency and to control composition of the syngas (with SN preferably being between 2.0 and 2.2 for subsequent use in methanol synthesis). An advantage of this scheme is that by mixing the GHR effluent with pre-reformed gas feed for the ATR, the methane content in the GHR effluent is significantly lowered; which adds to reducing the methane content of the final syngas.

In an especially preferred embodiment of the process according to the invention, the heated pre-reformed gas stream is divided into three streams that are fed to SMR, GHR and ATR reactors operated in parallel, with SMR and GHR reactors also being operated in series with the ATR. The syngas stream leaving the ATR is first used to heat the GHR, and then further cooled, and compressed in case of subsequent methanol synthesis steps.

Preferably, about 25-30 vol% of the pre-reformed stream is fed to the SMR, about 15-30, more preferably 15-20 vol% is fed to the GHR, and the effluent streams from the SMR and GHR units and about 40-55, more preferably 45-55, even more preferably 50-55 vol% of the pre-reformed gas stream are mixed and fed to the ATR, to optimise process efficiency and to control composition of the syngas (with SN preferably being between 2.0 and 2.2 for subsequent use in methanol synthesis). An advantage of this process scheme is high methane conversion; that is a very low concentration of methane in the produced syngas. This has the additional advantage in methanol production that less inert gas components are present in the methanol synthesis loop, resulting in improved conversion of syngas to methanol. A further advantage of this scheme is that lower gaseous feedstock and syngas flow rates, including less steam, are needed to obtain a certain methanol production rate; which reduces the volume of gas to be compressed and thus improves overall energy consumption of the methanol process.

In the process according to the invention at least one unit of each of the 4 different reactors is used. Although more than one reactor of a certain type may be applied, the process preferably uses one unit of each reactor, in view of plant complexity and investment costs.

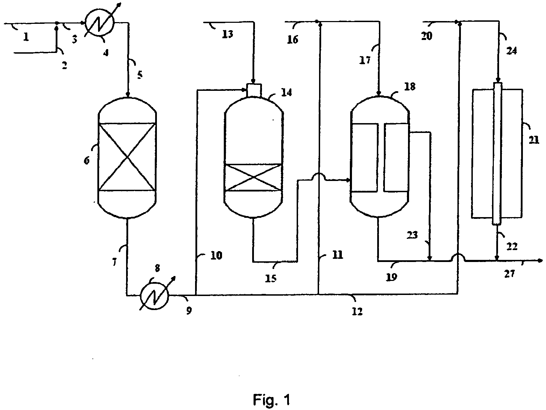

The above discussed process schemes are further illustrated in Figures 1-3, providing simplified process flow diagrams of 3 embodiments of the process according to the invention. In these figures, same reference numbers have the same or similar meaning; as further elucidated in the descriptions below.

In each of the Figures a desulphurised methane-rich gas stream **1** is mixed with a steam stream **2** at a pressure of about 3-4 MPa and a temperature of about 350-370 °C, at a steam to carbon ratio of about 0.5-3.0. This mixed stream **3** is then heated to about 500-550 °C in heat exchange coil **4**, which coil is installed in the convection duct of steam reformer **21** or in the effluent stream of ATR **14**. Preheated mixed stream 5 is then passed through a conventional adiabatic pre-reformer **6**, which contains Ni-based catalyst. The pre-reformed stream **7** leaves the APR at about 440-500°C, and is heated to about 600-650°C in heat exchanger **8** (also installed in the convection duct of steam reformer **21** or in the effluent stream of the ATR).

Referring now to the flow diagram of Figure 1, the reheated stream **9** is divided into three streams **10, 11** and **12**. These three streams are reformed in parallel in ATR **14**, GHR **18** and SMR **21**. Stream **10** is about 60-65 % of the pre-reformed stream **9**, and is mixed with preheated oxygen stream **13** and then passed through the burner installed in ATR **14**. This stream undergoes partial oxidation in the combustion zone, and unreacted methane in the hot combustion gases is subsequently steam reformed over the Ni-based catalyst bed. The effluent stream **15** leaves the ATR at about 885-1000 °C. The second divided stream **11,** forming about 5-15 % of stream **9,** is mixed with additional steam via stream **16,** and the mixed stream **17** is fed to GHR **18**. A steam to carbon ratio of 2.5-2.8 is maintained in stream **17**. The stream **17** is passed over the catalyst filled tubes in GHR **18** and the methane in this stream is steam reformed. The effluent stream **15** from ATR **14** is passed through shell side of the GHR **18** to supply required heat for this reforming reaction. The effluent stream **19** leaves GHR **18** leaves at 860-880 °C. The third divided stream **12,** which is about 30-35% of stream **9,** is mixed with additional steam via stream **20** to maintain a steam to carbon ratio of 2.5-2.8 in mixed stream **24**. This mixed stream **24** is steam reformed in conventional SMR **21**. The effluent stream **22** leaves the SMR **21** at about 860-880°C. The three reformed streams **22, 19** and **23** are mixed to form a total synthesis gas stream **27**. After heat recovery and cooling the stream is sent to compression and methanol synthesis units.

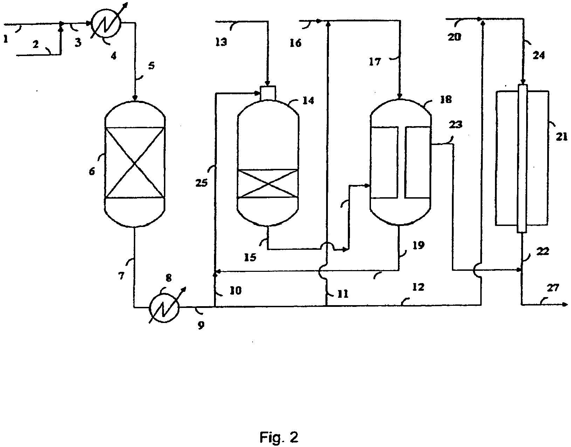

In the flow diagram presented in Figure 2, the reheated stream 9 is divided into three streams **10, 11** and **12;** of which stream **10** forms about 45-50 %, stream **11** is about 20-30% and stream **12** is about 25-30% of stream **9**. The stream **11** is passed over the Ni-based catalyst filled tubes in GHR **18,** wherein the gas feed is partially steam reformed. Additional steam may also be added to stream **11** via stream **16** for adjustment of steam to carbon ratio. The effluent stream **19** leaves GHR **18** at about 750-775 °C. Stream **10** is mixed with the effluent stream **19** from GHR **18,** and mixed stream **25** is fed to ATR **14**. A preheated oxygen stream 13 is also fed to ATR **14**. The streams **25** and **13** are passed through a burner installed in ATR **14** and partial combustion of methane with oxygen takes place in a combustion zone above a catalyst bed. The hot partial combustion products are then passed over the Ni-based catalyst bed, and remaining methane is steam reformed. The effluent stream 15 leaves the ATR **14** at about 985-1000 °C, and is then passed through the shell side of the GHR **18** to transfer the heat required for reforming reactions in GHR **18**. The stream **12** is sent to SMR **21,** optionally with additional steam **20** to adjust the steam to carbon ratio in feed stream **24** before feeding to SMR **21**. The effluent stream **22** leaves the SMR **21** at 850-880 °C, and is mixed with stream **23** from shell side of GHR **18**. The mixed stream **27,** forming the total synthesis gas stream, is sent for compression and methanol synthesis unit after heat recovery and cooling.

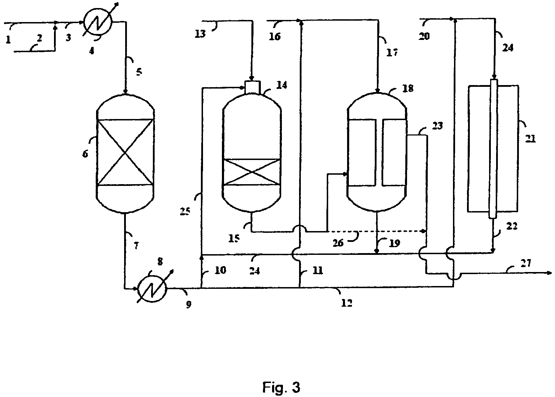

In the flow diagram of Figure 3, the reheated stream **9** is divided into three streams **10, 11** and **12;** of which stream **10** forms about 50-55%, stream **11** about 15-20% and stream 12 about 25-30% of stream **9**. Streams **11** and **12** are steam reformed in parallel in GHR **18** and SMR **21,** respectively Additional steam can also be added to stream **11** and/or stream **12** via stream **16** and stream **20,** respectively, to adjust the steam to carbon ratio at 2.5-2.8 in the inlet feed streams. The effluent stream **19** leaves GHR **18** at 800-850 °C, and effluent stream **22** from SMR 21 leaves at about 850-870 °C. Both streams are combined and mixed with stream **10,** and the mixed stream **25** is sent to ATR **14**. Stream **25** and preheated oxygen stream **13** are passed through the burner installed in ATR **14,** and undergo partial oxidation. The hot combustion gases are subsequently reformed over a Ni-based catalyst bed. The effluent stream **15** leaves ATR **14** at 886-1000 °C, and is passed through the shell side of GHR **18** to supply heat required for the steam reforming reaction in the GHR. Part of the stream **15** can optionally be bypassed via stream **26**. The stream **23** leaving the shell side of GHR **18,** optionally mixed with bypass stream **26,** forms the total syngas stream **27**. After heat recovery and cooling this stream is sent to compression and methanol synthesis units.

In the process according to the invention the gas distribution rates, and operating conditions of the reformers are adjusted, within above indicated ranges, such that the composition of the syngas product stream is suitable for subsequent use in methanol synthesis; i.e. preferably the syngas mixture has a SN of 2.0-2.2, more preferably SN is 2.0-2.1.

The invention relates to an integrated process for making methanol from a methane-rich gaseous feedstock comprising the combined reforming process according to the invention, with preferred process schemes and conditions as described above. In such a process following process steps can be distinguished: (a) syngas production, (b) methanol synthesis, and (c) methanol distillation. In the methanol process according to the invention known processes can be applied for steps (b) and (c); as for example described in the above referenced chapter in Ullmann's Encyclopedia of Industrial Chemistry.

In the integrated methanol process according to the invention, there can be various couplings between the steps (a), (b) and (c) to optimize consumption of feedstock and energy. Examples hereof include use of hot boiler water from syngas steps in methanol synthesis, recirculating unreacted syngas components from methanol synthesis back to methanol synthesis loop or using it as fuel for the SMR.

The present invention is now further elucidated with the following experiments, which include several process simulations of the schemes depicted in Figures 1-3.

**Examples**

In the following production of methanol from a methane-rich gas with a process according to the various flow schemes is simulated using a standard simulation package, such as Pro-II, taking into account boundary conditions for the reforming reactors as described in the above:

- SMR unit: maximum heat load about 1150 GJ/h;
- GHR unit: maximum energy input 420 GJ/h;
- ATR unit: limited by maximum ASU capacity of about 5200 kmol/h of oxygen.

Table 2 shows the simulation results for a methanol production capacity of 10000 mtpd based on the flow rates, compositional data, temperatures and pressures as provided by US 6100303 in Examples 1-3, Comparative examples 1-2, and Tables 1-5; now re-named as Comparative experiments A-E, respectively. The composition of the natural gas used as feed in the experiments is given in Table 1.

Table 1

| Component | Composition of natural gas feedstock (vol%) | | |
|---|---|---|---|
| | Comp. exp. A | Comp. exp. B-E | Examples 1-3 |
| $CH_4$ | 98.5 | 95.60 | 89.54 |
| $C_2H_6$ | 1.0 | 3.39 | 3.91 |
| $C_3H_8$ | 0.5 | 0.09 | 0.48 |
| $C_4H_{10}$ | | 0.18 | 0.24 |
| $C_5H_{12}$ | | | 0.08 |
| CO | | | 0.00 |
| $CO_2$ | | 0.47 | 0.30 |
| $N_2$ | | 0.26 | 5.45 |
| $H_2O$ | | 0.01 | |
| total | 100 | 100 | 100 |

The resulting data as listed in Table 2 are in good agreement with figures reported in US6100303, e.g. in Table 6 therein. It is noted that indicated heat load is the heat required for carrying out the reforming reaction, not the fuel requirement. Carbon efficiency is defined as the total molar flow rates of CO and CO2 converted to methanol relative to the total molar flow rates of CO and CO2 in the syngas.

From these data it can be concluded that in all experiments according to the process as disclosed in US 6100303 either SMR and/or GHR units are to be used that have an energy requirement exceeding practical limitations for such equipment as discussed hereinabove. In CE D also more oxygen is needed than can be supplied from an available ASU. Said critical figures are indicated in italics.

In Table 3 results are given as Examples 1-3 for simulations based on the process flow schemes as discussed above and shown schematically in Figures 1-3. In these simulations a final syngas stream with SN 2.18 is made from a methane-rich natural gas of composition given in Table 1, to enable subsequent methanol with a production capacity of 10000 mtpd, utilising the maximum capacity of available SMR, GHR and ASU single line equipment.

It can be seen that for each of the flow schemes according to the process of the invention the SMR and GHR units can be operated below their maximum feasible capacities; meaning that methanol production rates of over 10000 mtpd are possible with some adjustment of for example flow distribution rates and reactor conditions. Alternatively, smaller reactors units may be employed in a single line plant of 10000 mtpd capacity, thus further lowering investment costs.

In Example 3 the final syngas stream, which can be used to result in the targeted methanol production rate, has lower methane content, and is of lower flow rate than in the other examples; whereas the maximum capacity of the SMR unit is not yet fully used, resulting in high overall production efficiency.

Table 2

| | unit | CE A | CE B | CE C | CE D | CE E |
|---|---|---|---|---|---|---|
| | | Based on US 6100303 data listed in | | | | |
| | unit | Table 1 | Table 2 | Table 3 | Table 4 | Table 5 |
| | | | | | | |
| Total gaseous feed rate | kmol/h | 13493 | 13236 | 12974 | 12795 | 132236 |
| Gaseous feed pressure | MPa | 2.7 | 2.7 | 2.7 | 2.7 | 2.7 |
| **SMR unit:** | | | | | | |
| Mixed gas feed rate | kmol/h | 26046 | 27904 | 14259 | 1875 | 47538 |
| Steam to Carbon ratio | | 2.5 | 2.5 | 2.5 | 2.5 | 2.5 |
| Gas inlet temperature | °C | 560 | 560 | 560 | 560 | 560 |
| Gas exit temperature | °C | 800 | 800 | 800 | 800 | 777 |
| Methane leakage | vol% | 10.6 | 10.6 | 10.6 | 10.6 | 9.1 |
| Heat load | GJ/h | *1336* | *1421* | 726 | 95 | 2583 |
| **GHR unit:** | | | | | | |
| Mixed gas feed rate | kmol/h | 21853 | 19633 | 32339 | 44078 | |
| Steam to Carbon ratio | | 2.5 | 2.5 | 2.5 | 2.5 | |
| Gas inlet temperature | °C | 340 | 360 | 360 | 340 | |
| Gas exit temperature | °C | 819 | 846 | 726 | 660 | |
| Methane leakage | Vol% | 9.5 | 7.0 | 20.8 | 31.4 | |
| Heat load | GJ/h | *1400* | *1380* | *1392* | *1413* | |
| **ATR unit:** | | | | | | |
| Mixed gas feed rate | kmol/h | 66340 | 66600 | 60450 | 58050 | 66603 |
| Oxygen feed rate | kmol/h | 3233 | 2955 | 4583 | 6193 | 2955 |
| Steam to Carbon ratio | | 4.7 | 5.0 | 3.5 | 2.9 | 5.0 |
| Gas inlet temperature | °C | 809 | 819 | 749 | 673 | 777 |
| Gas exit temperature | °C | 975 | 975 | 975 | 975 | 975 |
| | | CE A | CE B | CE C | CE D | CE E |
| | | Based on US 6100303 data listed in | | | | |
| | unit | Table 1 | Table 2 | Table 3 | Table 4 | Table 5 |
| Methane leakage | vol% | 0.6 | 0.6 | 0.4 | 0.3 | 0.6 |
| Heat loss | GJ/h | 116 | 105 | 171 | 226 | 93 |
| | | | | | | |
| Total syngas flow rate (dry) | kmol/h | 51383 | 51283 | 47750 | 44525 | 51285 |
| SN | | 2.50 | 2.50 | 2.25 | 2.00 | 2.50 |

(continued)

|  |  | CE A | CE B | CE C | CE D | CE E |
|---|---|---|---|---|---|---|
|  |  | Based on US 6100303 data listed in | | | | |
|  | unit | Table 1 | Table 2 | Table 3 | Table 4 | Table 5 |
|  |  |  |  |  |  |  |
| Carbon efficiency | % | 96.7 | 96.9 | 98.2 | 99.0 | 96.9 |
| Methanol production rate | mtpd | 10000 | 10000 | 10000 | 10000 | 10000 |

Table 3

|  |  | Example 1 | Example 2 | Example 3 |
|---|---|---|---|---|
|  | unit |  |  |  |
| Total gaseous feed rate | kmol/h | 15653 | 15074 | 13999 |
| Gaseous feed pressure | MPa | 4.1 | 4.1 | 4.1 |
| **APR unit:** |  |  |  |  |
| Mixed gas feed rate | kmol/h | 47594 | 53303 | 42418 |
| Steam to Carbon ratio |  | 2.0 | 2.5 | 2.0 |
| Gas inlet temperature | °C | 500 | 500 | 500 |
| Gas exit temperature | °C | 448 | 446 | 446 |
| **SMR unit:** |  |  |  |  |
| Mixed gas feed rate | kmol/h | 17311 | 15146 | 14933 |
| Steam to Carbon ratio |  | 2.5 | 2.5 | 2.5 |
| Gas inlet temperature | °C | 617 | 650 | 616 |
| Gas exit temperature | °C | 870 | 870 | 870 |
| Methane leakage | vol% | 8.85 | 8.70 | 8.31 |
| Heat load | GJ/h | 887 | 741 | 774 |
| **GHR unit:** |  |  |  |  |
| Mixed gas feed rate | kmol/h | 4172 | 13804 | 9274 |
| Steam to Carbon ratio |  | 2.5 | 2.5 | 2.5 |
| Gas inlet temperature | °C | 616 | 650 | 616 |
| Gas exit temperature | °C | 870 | 750 | 832 |
| Methane leakage | Vol% | 9.1 | 21.3 | 11.1 |
| Heat load | GJ/h | 213 | 364 | 418 |
| **ATR unit:** |  |  |  |  |
| Mixed gas feed rate | kmol/h | 30711 | 43065 | 55701 |
|  |  | **Example 1** | **Example 2** | **Example 3** |
|  | unit |  |  |  |
| Oxygen feed rate | kmol/h | 5208 | 5208 | 5208 |
| Steam to Carbon ratio |  | 2.0 | 2.7 | 2.7 |
| Gas inlet temperature | °C | 650 | 685 | 757 |

(continued)

| | unit | Example 1 | Example 2 | Example 3 |
|---|---|---|---|---|
| Gas exit temperature | °C | 985 | 985 | 987 |
| Methane leakage | vol% | 0.98 | 0.73 | 0.84 |
| Heat loss | GJ/h | 0 | 0 | 0 |
| | | | | |
| Total syngas flowrate (dry) | kmol/h | 51125 | 50791 | 48949 |
| Methane leakage (overall) | vol% | 4.08 | 2.93 | 0.84 |
| SN | | 2.18 | 2.18 | 2.18 |
| | | | | |
| Carbon efficiency | % | 95 | 95 | 95 |
| Methanol production rate | mtpd | 10000 | 10000 | 10000 |

**Claims**

1. Process for making methanol from a synthesis gas mixture, said process comprising a combined reforming process for making the synthesis gas mixture from a desulphurised methane-rich gaseous feedstock containing at least 90 mol% methane, wherein the gaseous feedstock is mixed with steam and passed through an adiabatic pre-reformer (APR), and wherein pre-reformed gas from the APR is divided into three streams that are fed to a steam methane reformer (SMR), a gas heated reformer (GHR) and to an auto-thermal reformer (ATR), which reforming reactors are operated in parallel.

2. Process according to claim 1, wherein pre-reformed gas from the APR is heated to a temperature of about 600-650 °C.

3. Process according to any one of claims 1-2, wherein 35-65 vol% of the pre-reformed gas stream is fed to the ATR.

4. Process according to any one of claims 1-2, wherein the SMR and GHR units are also operated in series with the ATR.

5. Process according to claim 4, wherein effluent streams from the SMR and GHR and about 45-55 vol% of the prereformed gas stream from the APR are mixed and fed to the ATR.

6. Process according to any one of claims 1-5, wherein the synthesis gas mixture has a stoichiometric number (SN) of 2.0-2.2.

**Patentansprüche**

1. Verfahren zur Herstellung von Methanol aus einer Synthesegasmischung, wobei das Verfahren einen kombinierten Reformierungsprozess umfasst für die Herstellung der Synthesegasmischung aus einem entschwefelten Methan-reichen gasförmigen Ausgangsmaterial enthaltend mindestens 90 Mol% Methan, worin das gasförmige Ausgangsmaterial mit Dampf gemischt wird, und durch einen adiabatischen Pre-Reformer (APR) hindurchgeführt wird, und worin pre-reformiertes Gas aus dem APR in drei Ströme aufgeteilt wird, die zu einem Dampf-Methan-Reformer (SMR), einem Gas-beheizten Reformer (GHR) und zu einem auto-thermischen Reformer (ATR) geführt werden, wobei die Reformerreaktoren parallel betrieben werden.

2. Verfahren gemäß Anspruch 1, worin pre-rreformiertes Gas von dem APR auf eine Temperatur von ungefähr 600-650°C erhitzt wird.

3. Verfahren gemäß irgendeinem der Ansprüche 1-2, worin 35-65 Volumen% des pre-reformierten Gasstroms zu dem ATR geführt wird.

**4.** Verfahren gemäß irgendeinem der Ansprüche 1-2, worin die SMR- und GHR-Einheiten auch in Serie mit dem ATR betrieben werden.

**5.** Verfahren gemäß Anspruch 4, worin Abflussströme von dem SMR und GHR und ungefähr 45-55 Volumen% des pre-reformierten Gasstroms von dem APR gemischt werden, und zu dem ATR geführt werden.

**6.** Verfahren gemäß irgendeinem der Ansprüche 1-5, worin die Synthesegasmischung eine stöchiometrische Zahl (SN) von 2,0-2,2 hat.

**Revendications**

**1.** Procédé de fabrication de méthanol à partir d'un mélange de gaz de synthèse, ledit procédé comprenant un procédé de reformage combiné pour fabriquer le mélange de gaz de synthèse à partir d'une matière première gazeuse riche en méthane désulfurée contenant au moins 90 % en moles de méthane, dans lequel la matière première gazeuse est mélangée avec de la vapeur et passée à travers un pré-reformeur adiabatique (APR), et dans lequel le gaz pré-reformé provenant de l'APR est divisé en trois flux qui sont alimentés vers un reformeur de méthane-vapeur (SMR), un reformeur de gaz chauffé (GHR) et un reformeur auto-thermique (ATR), lesdits réacteurs de reformage étant utilisés en parallèle.

**2.** Procédé selon la revendication 1, dans lequel le gaz pré-reformé provenant de l'APR est chauffé à une température d'environ 600 à 650 °C.

**3.** Procédé selon l'une quelconque des revendications 1 à 2, dans lequel 35 à 65 % en volume du flux de gaz pré-reformé est alimenté vers l'ATR.

**4.** Procédé selon l'une quelconque des revendications 1 à 2, dans lequel les unités SMR et GHR sont également utilisées en série avec l'ATR.

**5.** Procédé selon la revendication 4, dans lequel les flux d'effluent provenant du SMR et du GHR et environ 45 à 55 % en volume du flux de gaz pré-reformé provenant de l'APR sont mélangés et alimentés vers l'ATR.

**6.** Procédé selon l'une quelconque des revendications 1 à 5, dans lequel le mélange de gaz de synthèse a un nombre stoechiométrique (SN) de 2,0 à 2,2.

Fig. 1

Fig. 2

Fig. 3

## REFERENCES CITED IN THE DESCRIPTION

**Patent documents cited in the description**

- US 6100303 A
- EP 0989094 A2
- WO 9315999 A
- US 4999133 A
- US 5512599 A
- US 5496859 A
- EP 0522744 A2
- US 20040063797 A1
- EP 1403216 A1
- GB 2407819 A
- EP 1241130 A1
- EP 0440258 A2
- EP 0959120 A1

**Non-patent literature cited in the description**

- Synthesis Gas Generation: Industrial'' of the ''Encyclopedia of Catatysis. **P. F. VAN DEN.** Oosterkamp in chapter. John Wiley & Sons, 13 December 2002
- Ullmann's Encyclopedia of Industrial Chemistry. Wiley-VCH Verlag, 15 June 2000